# EUROPEAN PATENT APPLICATION

(11) **EP 2 857 387 A1**
(43) Date of publication of application: **08.04.2015**
(21) Application number: 13187506.4
(22) Date of filing: 07.10.2013
(51) Int. Cl.: C07D 237/24

(54) **Process for manufacturing 1,6-dihydro-6-oxo-4-pyridazine carboxylic acid**

(71) Applicant: BOEHRINGER INGELHEIM INTERNATIONAL GMBH, 55216 Ingelheim am Rhein (DE)
(72) Inventor: Linz, Guenter, 88441 Mittelbiberach (DE); Kraemer, Friedhelm, 88436 Eberhardzell (DE); Schnaubelt, Juergen, 88447 Oberhoefen / Warthausen (DE); Stehle, Emanuel, Biberach an der Riss (DE)
(74) Representative: Simon, Elke Anna Maria

(57) **Abstract**

The present invention provides a process for manufacturing 6-oxo-1 H-pyridazine-4-carboxylic acid 5 comprising - (a) reacting dimethyl 2-methylenebutanedioate with hydrazine to obtain methyl 6- oxohexahydropyridazine-4-carboxylate 11; - (b) oxidizing methyl 6-oxohexahydropyridazine-4-carboxylate 11 with a suitable oxidizing agent to methyl 6-oxo-1 H-pyridazine-4-carboxylate 4a; - (c) isolating methyl 6-oxo-1 H-pyridazine-4-carboxylate 4a; - (d) hydrolyzing methyl 6-oxo-1 H-pyridazine-4-carboxylate 4a in the presence of an aqueous base or acid to the 6-oxo-1 H-pyridazine-4-carboxylic acid 5.

## Description

### FIELD OF THE INVENTION

This invention relates to a novel process for making 6-oxo-1H-pyridazine-4-carboxylic acid **5**.

### BACKGROUND OF THE INVENTION

The first synthesis [McMillan et al. JACS 1955, 77, 3376-8] of 6-oxo-1H-pyridazine-4-carboxylic acid **5** starts from diethyl 2-formylbutanedioate **1** and hydrazine hydrate. In the first step, a mixture of ethyl 2-(5-oxo-1,4-dihydropyrazol-4-yl)acetate **2** and ethyl 6-oxo-4,5-dihydro-1H-pyridazine-4-carboxylate **3** is obtained. The 6-oxo-4,5-dihydro-1H-pyridazine **3** is the minor product and is obtained after destillation as an oil with low yield in impure form. Dehydrogenation of **3** with bromine in acetic acid yields the ethyl 6-oxo-1H-pyridazine-4-carboxylate **4b.** The ethyl ester in **4b** is hydrolyzed to yield the 6-oxo-1H-pyridazine-4-carboxylic acid **5.**

Overall the low yield of 6-oxo-4,5-dihydro-1H-pyridazine **3** combined with the difficult purification of **3** are the major drawbacks of this synthetic approach.

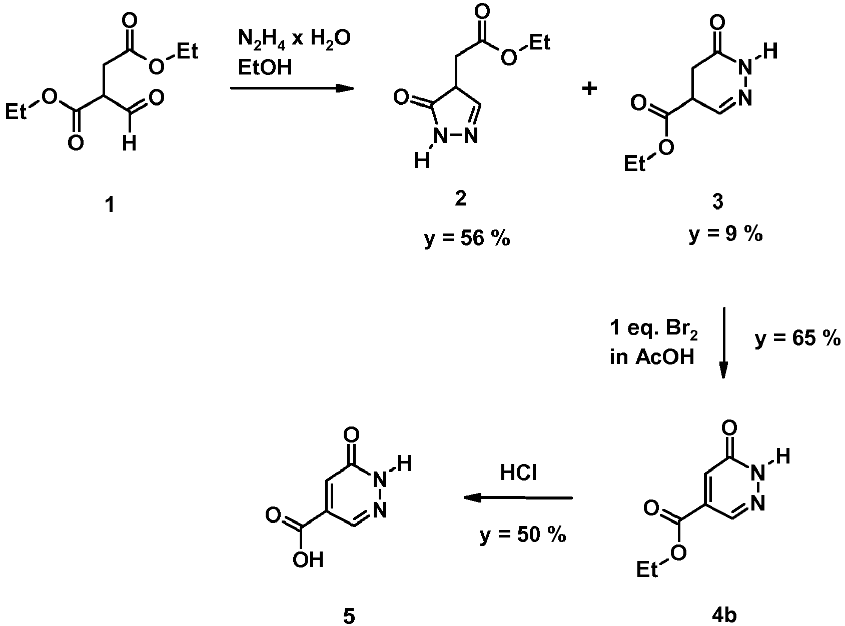

The dehydrogenation of a 4,5-dihydro-1H-pyridazine **6** (already containing one double bond) with bromine in acetic acid to the aromatic heterocycle **7** was first described by O. Poppenberg [Ber., 1901, 34, 3257].

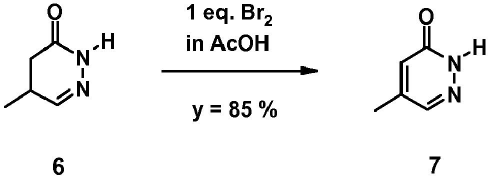

A short synthesis of **5** is described by B. Jägersten [B. Jägersten, Arkiv för Kemi 1969, 30, 261-267]. The key step of this approach is the nucleophilic addition of hydrazine upon the unsubstituted α-carbon of the furan ring followed by ring closure and oxidation to **4**b.

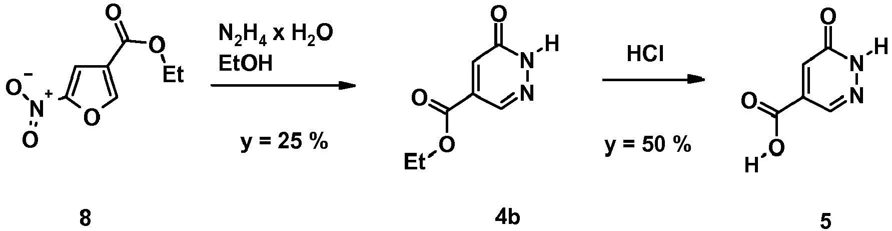

The disadvantage of the Jägersten's synthesis lies in the low yields of 4b in the first step and formation of substantial quantities of side products. Besides, the starting material 8 is rather expensive.

WO 2009054791, WO 2009054794, WO2009054793, WO2009054789 and US20070259862 disclose another method for the manufacture of compound 5 starting from ethyl (E)-4,4-dimethoxy-3-methyl-but-2-enoate **9**. The latter can be obtained in a Horner-Emmons-Wadsworth reaction [E.J. Corey et al., J. Am. Chem. Soc., 2004, 126, 5984].

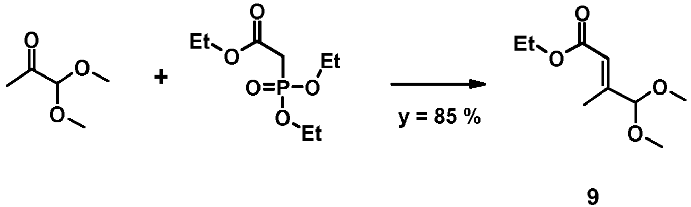

Compound **9** is then treated directly with hydrazine hydrate and in turn with acid to yield the 4-methyl substituted 1H-pyridazin-6-one **(7).** Oxidation of compound 7 with potassium dichromate yields 6-oxo-1H-pyridazine-4-carboxylic acid **5.**

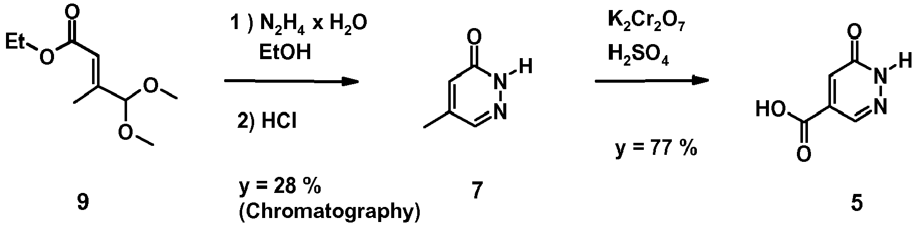

Low yield in the cyclization step and the highly toxic potassium dichromate render this method industrially impracticable.

WO2008041075 and US20070259860 disclose a slightly improved process starting from compound **9.** The latter is cyclized to 2-hydroxy-3-methyl-2H-furan-5-one **10** and then converted with hydrazine hydrate to 4-methyl-1H-pyridazin-6-one **7.** The last oxidation step is conducted with potassium permanganate as oxidant.

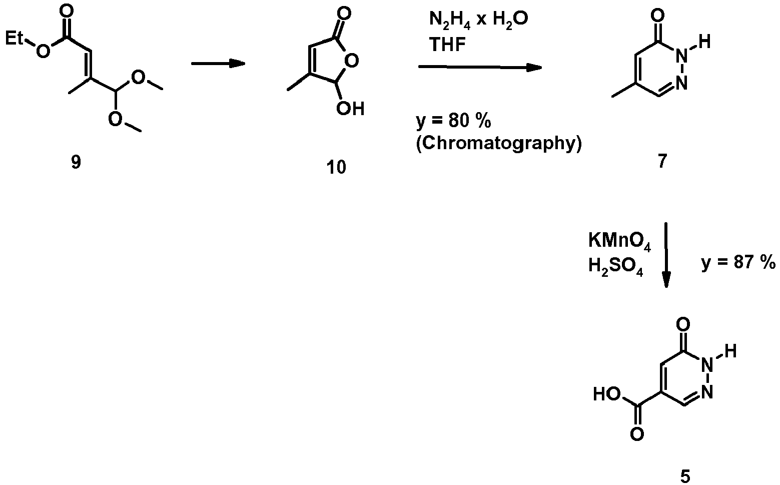

This method however still suffers from several drawbacks including 4 overall steps, chromatographic separation of compound **7,** harsh conditions for the oxidation step involving use of potassium permanganate with sulfuric acid, the latter requiring tedious work up. These conditions make it rather difficult to scale up this process.

### DETAILED DESCRIPTION OF THE INVENTION

The present invention provides a process for manufacturing 6-oxo-1H-pyridazine-4-carboxylic acid **5** comprising
(a) reacting dimethyl 2-methylenebutanedioate with hydrazine to obtain methyl 6-oxohexahydropyridazine-4-carboxylate **11;**
(b) oxidizing methyl 6-oxohexahydropyridazine-4-carboxylate **11** with a suitable oxidizing agent to methyl 6-oxo-1H-pyridazine-4-carboxylate **4a;**
(c) isolating methyl 6-oxo-1H-pyridazine-4-carboxylate **4a;**
(d) hydrolyzing methyl 6-oxo-1H-pyridazine-4-carboxylate **4a** in the presence of an aqueous base or acid to the 6-oxo-1H-pyridazine-4-carboxylic acid **5.**

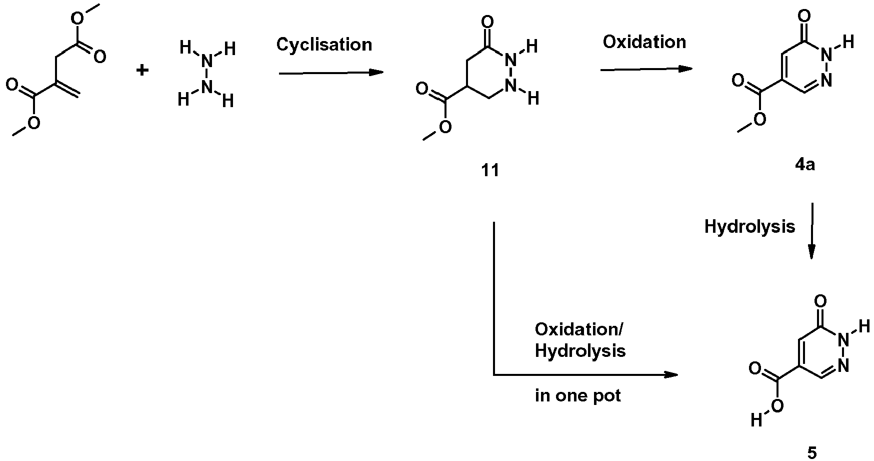

The process according to the present invention overcomes the disadvantages of processes of the prior art by having the distinction of
1) making use of inexpensive and readily accessible starting material and reagents,
2) consisting of 2-3 chemical steps only that can easily be carried out on industrial scale,
3) yielding 6-oxo-1H-pyridazine-4-carboxylic acid in pure form with high overall yield.

According to a second embodiment of the present invention, step (c) is omitted. The hydrolysis according to step (d) is carried out in the reaction mixture resulting in step (b) under acidic conditions or by adding the aqueous base to the reaction mixture obtained in step (b).

In another embodiment, in step (a), dimethyl 2-methylenebutanedioate is reacted at a temperature of 0-60°C with 0.9-1.5 molar equivalents (eq.) of hydrazine hydrate.

In a further embodiment, in step (a), dimethyl 2-methylenebutanedioate is reacted at a temperature of 0-10°C with 1-1.1 molar equivalents (eq.) of hydrazine hydrate.

In a further embodiment, in step (b), the oxidizing agent is selected from the group consisting of bromine, chlorine, a mixture of hydrogen peroxide and hydrobromic acid, 1,3-dichloro-5,5-dimethylhydantoin, 1,3-dibromo-5,5-dimethylhydantoin, N-chlorosuccinimide, N-bromosuccinimide, chloranil (2,3,5,6-tetrachloro-1,4-benzoquinone), DDQ (2,3-dichloro-5,6-dicyano-1,4-benzoquinone), sodium hypochlorite and sodium hypobromite. In a further embodiment, in step (b), the oxidation is carried out with 2-3 eq. of bromine at a temperature not exceeding 50°C.

In a further embodiment, in step (b), the oxidation is carried out with 2-2.5 eq. of bromine at a temperature not exceeding 25°C.

In a further embodiment, in step (b), the oxidation is carried out with a mixture of 2-4 eq. of hydrogen peroxide and 0,5-8 eq. of hydrobromic acid.

In a further embodiment, in step (d), methyl 6-oxo-1H-pyridazine-4-carboxylate **4a** is hydrolyzed in the presence of 1-10 eq. of an aqueous alkali base.

In a further embodiment, in step (d), methyl 6-oxo-1H-pyridazine-4-carboxylate **4a** is hydrolyzed in the presence of 1-2 eq. of aqueous sodium hydroxide.

### EXPERIMENTAL SECTION

### Synthesis of 6-oxo-1H-pyridazine-4-carboxylic acid (5)

**Step 1:** methyl 6-oxohexahydropyridazine-4-carboxylate [see also: J. Chem. Soc., Perkin Trans. 1, Org. Bioorg. Chem. 1993, 16, 1931]

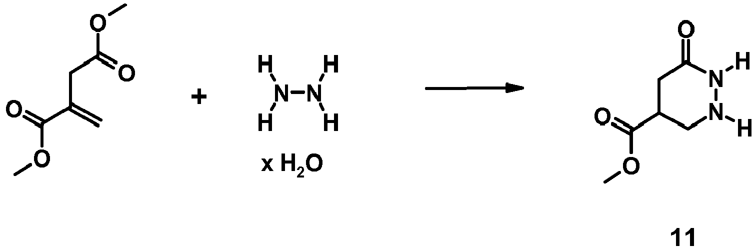

A suspension of 50 g dimethyl 2-methylenebutanedioate in 300 mL 2-propanol is cooled in an ice bath. Hydrazine hydrate (16.6 g) was added over 15 minutes. The reaction mixture was stirred for 6 hours at 0 to 5 °C. The precipitated product was filtered by suction, washed with 2-propanol and dried under reduced pressure.

Yield: 39.1 g (78 % of theory) of methyl 6-oxohexahydropyridazine-4-carboxylate **11** as colorless crystalline solid.

¹H NMR (400 MHz, DMSO-d₆) δ (ppm) 8.85 (bs, 1H), 5.04 (dd, 1H), 3.63 (s, 3H), 3.20 - 3.11 (m, 1H), 3.09 - 3.00 (m, 1H), 2.93 - 2.83 (m, 1H), 2.50 - 2.38 (m, 2H).

**Step 2:** methyl 6-oxo-1H-pyridazine-4-carboxylate **4a**

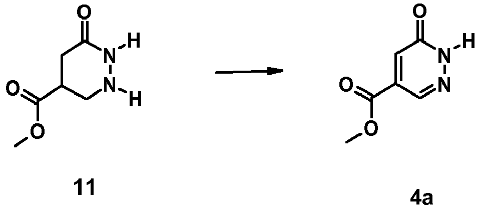

A solution of 5 g methyl 6-oxohexahydropyridazine-4-carboxylate 11 in 40 mL water was cooled in an ice bath. Bromine (10.5 g) was added so that the temperature does not exceed 25 °C. The colorless suspension was stirred for 16 hours. The precipitated product was filtered by suction, washed with water and dried under reduced pressure.

Yield: 3.95 g (81 % of theory) of methyl 6-oxo-1H-pyridazine-4-carboxylate **4a** as a colorless solid. ¹H NMR (400 MHz, DMSO-d₆) δ (ppm) 13.5 (bs, 1H), 8.15 (d, 1H, *J* = 1.98 Hz), 7.27 (d, 1H, *J* = 1.98 Hz), 3.89 (s, 3H).

**Step 3:** Hydrolysis 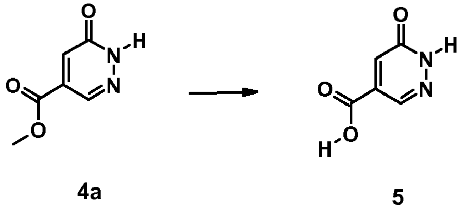 0.37 mL of a 4 molar aqueous sodium hydroxide solution was added to a solution of 150 mg of methyl 6-oxo-1H-pyridazine-4-carboxylate 4a in 1.5 mL methanol. The reaction mixture was stirred for 2 hours at 20 °C. 0.37 mL of a 4 molar hydrochloric acid are added. The precipitated product was filtered by suction, washed with water and dried at 50 °C

Yield: 90 mg (66 % of theory) of 6-oxo-1H-pyridazine-4-carboxylic acid **5** as a solid.

¹H NMR (400 MHz, DMSO-d₆) δ (ppm) 14.0 (bs, 1H), 13.4 (bs, 1H), 8.13 (d, 1H, J = 1.95 Hz), 7.23 (d, 1H, J = 1.95 Hz)

**One-pot Synthesis of 6-oxo-1H-pyridazine-4-carboxylic acid (5)** 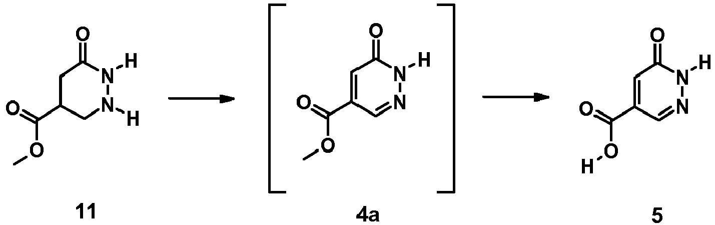 25.8 g of an aqueous hydrogen peroxide solution (35 %) were slowly added under cooling to a solution of 87.4 g hydrobromic acid (48 %) in 100 mL acetic acid so that the temperature did not exceed 15 °C. The solution was cooled to -10 °C. A solution of 20 g methyl 6-oxohexahydro-pyridazine-4-carboxylate 11 in 70 mL water was added over 15 minutes so that the temperature did not exceed -5 °C. The reaction mixture was stirred for 2 hours at 0 °C. Subsequently, the mixture was heated under reflux for 3.5 hours. The reaction mixture was cooled to 0 °C. The precipitated product was filtered by suction, washed with water and dried under reduced pressure at 50 °C.

Yield: 13.9 g (78 % of theory) of 6-oxo-1H-pyridazine-4-carboxylic acid **5** as a solid. ¹H NMR (400 MHz, DMSO-d₆) δ (ppm) 14.0 (bs, 1H), 13.4 (bs, 1H), 8.13 (d, 1H, J = 1.95 Hz), 7.23 (d, 1H, J = 1.95 Hz).

## Claims

1. Process for manufacturing 6-oxo-1H-pyridazine-4-carboxylic acid **5** 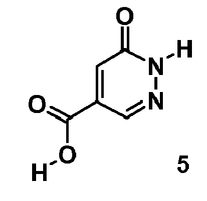 comprising
(a) reacting dimethyl 2-methylenebutanedioate 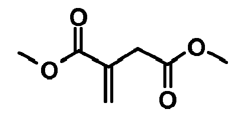 with hydrazine to obtain methyl 6-oxohexahydropyridazine-4-carboxylate **11** 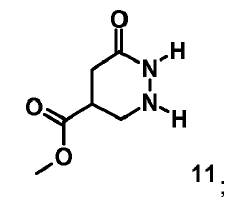
(b) oxidizing methyl 6-oxohexahydropyridazine-4-carboxylate **11** with a suitable oxidizing agent to methyl 6-oxo-1H-pyridazine-4-carboxylate **4a** 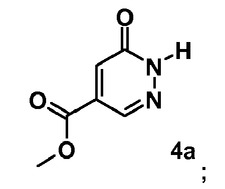
(c) isolating methyl 6-oxo-1H-pyridazine-4-carboxylate **4a;**
(d) hydrolyzing methyl 6-oxo-1H-pyridazine-4-carboxylate **4a** in the presence of an aqueous base or acid to the 6-oxo-1H-pyridazine-4-carboxylic acid **5.**

2. The process according to claim 1 **characterized in that** step (c) is omitted and the hydrolysis according to step (d) is carried out in the reaction mixture resulting in step (b) under acidic conditions or by adding the aqueous base to the reaction mixture obtained in step (b).

3. The process according to any one of the preceding claims **characterized in that** in step (a) the dimethyl 2-methylenebutanedioate is reacted at a temperature of 0-60°C with 0.9-1.5 eq. of hydrazine hydrate.

4. The process according to any one of the preceding claims **characterized in that** in step (a) the dimethyl 2-methylenebutanedioate is reacted at a temperature of 0-10°C with 1-1.1 eq. of hydrazine hydrate.

5. The process according to any one of the preceding claims **characterized in that** in step (b) the oxidizing agent is selected from the group consisting of bromine, chlorine, a mixture of hydrogen peroxide and hydrobromic acid, 1,3-dichloro-5,5-dimethylhydantoin, 1,3-dibromo-5,5-dimethylhydantoin, N-chlorosuccinimide, N-bromosuccinimide, chloranil (2,3,5,6-tetrachloro-1,4-benzoquinone), DDQ (2,3-dichloro-5,6-dicyano-1,4-benzoquinone), sodium hypochlorite and sodium hypobromite.

6. The process according to any one of the preceding claims **characterized in that** in step (b) the oxidation is carried out with 2-3 eq. of bromine at a temperature not exceeding 50°C.

7. The process according to any one of the preceding claims **characterized in that** in step (b) the oxidation is carried out with 2-2.5 eq. of bromine at a temperature not exceeding 25°C.

8. The process according to any one of the preceding claims **characterized in that** in step (b) the oxidation is carried out with a mixture of 2-4 eq. of hydrogen peroxide and 0,5-8 eq. of hydrobromic acid.

9. The process according to any one of the preceding claims **characterized in that** in step (d) the methyl 6-oxo-1H-pyridazine-4-carboxylate **4a** is hydrolyzed in the presence of 1-10 eq. of an aqueous alkali base.

10. The process according to any one of the preceding claims **characterized in that** in step (d) the methyl 6-oxo-1H-pyridazine-4-carboxylate **4a** is hydrolyzed in the presence of 1-2 eq. of aqueous sodium hydroxide.
